# EUROPÄISCHE PATENTSCHRIFT

(11) **EP 3 192 431 B1**
(45) Veröffentlichungstag und Bekanntmachung des Hinweises auf die Patenterteilung: **06.11.2024**
(21) Anmeldenummer: 17151105.8
(22) Anmeldetag: 12.01.2017
(51) Int. Cl.: A61B 3/103, A61B 3/032, A61B 3/00

(54) **SEHPRÜFSYSTEM UND VERFAHREN ZUM ÜBERPRÜFEN DER AUGEN**
VISION TESTING SYSTEM AND METHOD FOR TESTING EYES
SYSTÈME D'EXAMEN OPTOMÉTRIQUE ET PROCÉDÉ D'EXAMEN DES YEUX

(30) Priorität: 14.01.2016 DE 102016000232
(43) Veröffentlichungstag der Anmeldung: 19.07.2017
(73) Patentinhaber: Oculus Optikgeräte GmbH, 35582 Wetzlar (DE)
(72) Erfinder: STEINMÜLLER, Andreas, 35435 Wettenberg (DE); DEGLE, Prof. Dr. Stephan, 07743 Jena (DE)
(74) Vertreter: advotec.

(56) Entgegenhaltungen:
- JP-A- 2002 233 502
- US-A1- 2011 157 550
- US-A1- 2012 212 598

## Beschreibung

Die Erfindung betrifft ein Sehprüfsystem und ein Verfahren zum Überprüfen der Augen eines Probanden, aufweisend ein Anzeigegerät mit dem zumindest einem Auge des Probanden Sehprüfzeichen zur subjektiven Refraktionsmessung visualisierbar sind, wobei das Anzeigegerät eine Kameravorrichtung mit einer Kamera aufweist, mittels der Augen des Probanden aufnehmbar sind, wobei das Anzeigegerät eine Beleuchtungsvorrichtung mit einer Lichtquelle aufweist, mittels der der Augenhintergrund des Probanden beleuchtbar ist, wobei mittels der Kameravorrichtung des Anzeigegeräts eine Verteilung von Licht der Lichtquelle bei der Beleuchtung des Augenhintergrunds in der Pupille des Auges des Probanden aufnehmbar ist, wobei das Sehprüfsystem eine Steuervorrichtung aufweist, mittels der aus der Verteilung des Lichts der Lichtquelle in der Pupille eine objektive Refraktion des Auges bestimmbar ist, wobei das Anzeigegerät einen Bildschirm umfasst.

Derartige Sehprüfsysteme sind hinreichend bekannt und werden regelmäßig zur Durchführung von Sehtests genutzt. So verfügen die bekannten Anzeigegeräte beziehungsweise Bildschirme über ein Steuergerät, über welches eine Bedienperson eine Wiedergabe von Sehprüfzeichen auf dem Bildschirm steuern kann. Je nach Art des Bildschirms kann dieser auch über eine lineare oder eine zirkulare Polarisation verfügen. Die Polarisation des Bildschirms wird regelmäßig zur Durchführung von Sehtests in Verbindung mit einer Messbrille oder einem Phoropter genutzt. Die Kameravorrichtung kann beispielsweise zur Vermessung der Augen genutzt werden, wobei bei simulierten Dämmerungssehen oder Nachtsehen eine Vermessung aufgrund der Beleuchtungsbedingungen kaum möglich ist.

Bei den bekannten Sehprüfsystemen zur Bestimmung einer subjektiven Refraktion mittels Sehprüfzeichen ist es nachteilig, dass sich eine Bedienperson durch Darbietung verschiedenster Sehzeichen in Verbindung mit Probegläsern eines Phoropters oder einer Messbrille zunächst an die Refraktionswerte des Probanden iterativ herantasten beziehungsweise sich diesen nähern muss. Dieses Vorgehen nimmt regelmäßig relativ viel Zeit in Anspruch. Zwar können Refraktionswerte eines Probanden mithilfe eines Aberrometers oder Autorefraktometers auch objektiv bestimmt werden, jedoch unterscheiden sich möglicherweise die von dem Probanden subjektiv als optimal empfundenen Refraktionswerte von den objektiv ermittelten Refraktionswerten, weshalb die Bestimmung der subjektiven Refraktionswerte unerlässlich ist. Auch ist ein Aberrometer nicht immer vorhanden und auch vergleichsweise teuer, sodass die Bestimmung der subjektiven Refraktionswerte auch ohne eine vorherige Messung mit dem Aberrometer durchgeführt wird.

Aus der US 2012/212598 A1 ist ein Sehprüfsystem bekannt, welches eine Kamera und ein Feld mit in Reihen angeordneten Leuchtdioden umfasst. Vor einem Kameraobjektiv ist ein Teilerspiegel angeordnet, über den mit den Leuchtdioden einem Probanden konzentrische Kreise, oder andere die Aufmerksamkeit des Probanden erregende Lichtzeichen, dargestellt werden können. Weiter sind in dem Feld Infrarotleuchtdioden vorgesehen, die in einem Winkel, abweichend von der optischen Achse des Objektivs der Kamera, angeordnet sind, und mit denen eine Pupille eines Auges des Probanden beleuchtbar sein soll. Mit der Kamera wird dann ein Pupillenreflex des Infrarotlichts aufgenommen und eine objektive Refraktion des Auges des Probanden bestimmt. Unter anderem kann auch ein Gradient einer Helligkeitsverteilung eines Pupillenreflexes bestimmt werden. Weiter kann mittels der Leuchtdioden ein Fixaktionsreiz dargeboten werden, der dann eine Sehachse des Auges des Probanden relativ zu der optischen Achse des Objektivs der Kamera um einen Winkel verschieben soll.

Die US 2011/157550 A1 zeigt ein Sehprüfsystem, welches aus einem Bildschirm, einem Computer und einer Kamera bzw. Beleuchtungsvorrichtung gebildet ist. Die Beleuchtungsvorrichtung ist durch die IR-Leuchtdioden gebildet, mit denen Pupillen eines Probanden über einen Teilerspiegel mit Infrarotlicht beleuchtet werden können. Mittels einer Kamera können Pupillenreflexe aufgenommen und durch Photorefraktion eine objektive Refraktion bestimmt werden. Auf dem Bildschirm können Objekte zur Animation bzw. Fixation dargestellt werden. Auch kann vorgesehen sein, dass eine Allgemeinbeleuchtung des Raums mittels des Computers gesteuert wird. Diese Beleuchtung wird zur Erzeugung eines größeren Pupillendurchmessers genutzt.

Der vorliegenden Erfindung liegt daher die Aufgabe zugrunde, ein Sehprüfsystem und ein Verfahren zum Überprüfen der Augen eines Probanden mit einem Sehprüfsystem vorzuschlagen, mittels dem Sehtests schneller durchführbar sind.

Diese Aufgabe wird durch ein Sehprüfsystem mit den Merkmalen des Anspruchs 1 und ein Verfahren mit den Merkmalen des Anspruchs 9 gelöst.

Das erfindungsgemäße Sehprüfsystem zum Überprüfen der Augen eines Probanden umfasst ein Anzeigegerät, mit dem zumindest einem Auge des Probanden Sehprüfzeichen zur subjektiven Refraktionsmessung visualisierbar sind, wobei das Anzeigegerät eine Kameravorrichtung mit einer Kamera aufweist, mittels der Augen des Probanden aufnehmbar sind, wobei das Anzeigegerät eine Beleuchtungsvorrichtung mit einer Lichtquelle aufweist, mittels der der Augenhintergrund des Probanden beleuchtbar ist, wobei mittels der Kameravorrichtung des Anzeigegeräts eine Verteilung von Licht der Lichtquelle bei der Beleuchtung des Augenhintergrunds in der Pupille des Auges des Probanden aufnehmbar ist, wobei das Sehprüfsystem eine Steuervorrichtung aufweist, mittels der aus der Verteilung des Lichts der Lichtquelle in der Pupille eine objektive Refraktion des Auges bestimmbar ist, wobei das Anzeigegerät einen Bildschirm umfasst, wobei der Bildschirm geeignet ist, die Sehprüfzeichen für die subjektive Refraktionsmessung unmittelbar nach der Bestimmung der objektiven Refraktion durch die Steuervorrichtung zu visualisieren.

Mittels der Lichtquelle wird ein Augenhintergrund beziehungsweise eine Netzhaut des Auges des Probanden beleuchtet. Dabei ergibt sich bei einem fehlsichtigen Auge ein unscharfes Bild auf der Netzhaut des Auges. Dieses Bild wird durch die Pupille von der Kamera der Kameravorrichtung aufgenommen. Diese aufgenommene Lichtverteilung in der Pupille beziehungsweise Pupillenreflektion ist jeweils unterschiedlich in Abhängigkeit der vorliegenden Ametropie des jeweiligen Auges.

Die Steuervorrichtung beziehungsweise das Steuergerät ist vorzugsweise ein Computer, der die von der Kamera aufgenommene Lichtverteilung in der Pupille mittels Bildverarbeitung weiterverarbeitet. Die Steuervorrichtung kann so die Lichtverteilung in der Pupille bestimmen und beispielsweise mit einer normalen Lichtverteilung eines gesunden Auges beziehungsweise einer Vergleichsgruppe von Probanden vergleichen. Optional kann die Steuervorrichtung die Lichtverteilung in der Pupille des Auges des Probanden auch mit in einer Datenbank enthaltenen Lichtverteilungen von myopen Augen mit einem einfachen Bildvergleich vergleichen. So kann die Steuervorrichtung eine objektive Refraktion des Auges bestimmen beziehungsweise errechnen, die zwar nicht so genau ist wie eine Messung mit einem Aberrometer, jedoch bereits ausreichend genau ist, um einen subjektiven Sehtest mit Sehprüfzeichen in dementsprechend angepasster Größe durchführen zu können. Eine sonst erforderliche iterative Näherung an die subjektiven Refraktionswerte durch Darbietung verschiedenster Sehprüfzeichen wird folglich erheblich verkürzt. Weiter können mit ein und demselben Sehprüfsystem eine objektive Refraktion und eine subjektive Refraktion bestimmt werden, wodurch auf eine gesonderte Messung mit einem Aberrometer verzichtet werden kann. Das Sehprüfsystem ist somit auch kostengünstig zu betreiben.

Das Anzeigegerät, die Kamera und die Lichtquelle können in einem gemeinsamen Gehäuse des Sehprüfsystems angeordnet sein. Das Sehprüfsystem wird dann auch besonders einfach handhabbar, da nicht mehrere miteinander gekoppelte und dennoch voneinander beabstandete Geräte zur Durchführung eines Sehtestes benötigt werden.

Das Anzeigegerät kann ein ortsfestes Ferntest-Anzeigegerät sein, dessen Anzeigeflächengröße für Sehtests mit einem Sehabstand von 3 m bis 10 m, vorzugsweise von 4 m bis 8 m ausgebildet ist, und/oder ein mobil handhabbares Nahtest-Anzeigegerät sein, dessen Anzeigefläche größer für Sehtests mit einem Sehabstand von 10 cm bis 3 m, vorzugsweise von 30 cm bis 1 m ausgebildet ist. Das Ferntest-Anzeigegerät kann dann zur Darstellung von Sehprüfzeichen zum Testen des Fernsehens und das Nahtest-Anzeigegerät zur Darstellung von Sehprüfzeichen zum Testen des Nahsehens genutzt werden. Beide Anzeigegeräte können für sich alleine oder auch in Kombination miteinander als Sehprüfsystem verwendet werden. Das Ferntest-Anzeigegerät kann vorzugsweise in dem oben angegebenen Sehabstand relativ zu dem Probanden ortsfest aufgestellt oder an einer Wand montiert sein. Wenn der Proband relativ zum Ferntest-Anzeigegerät in einer definierten Sehentfernung zur Durchführung von Sehtests platziert wird, kann die Sehentfernung zu dem Ferntest-Anzeigegerät genau bestimmt werden. Auch kann dann eine Anzeigeflächengröße des Ferntest-Anzeigegerätes um ein Vielfaches größer sein als eine Anzeigeflächengröße des Nahtest-Anzeigegeräts, da gegebenenfalls auf der Anzeigefläche des Ferntest-Anzeigegeräts vergleichsweise größere Sehprüfzeichen dargestellt werden. Das Nahtest-Anzeigegerät kann auch mobil handhabbar sein, sodass dieses in einem nahezu beliebigen Abstand innerhalb des oben angegebenen Sehabstands relativ zu den Augen des Probanden von einer Bedienperson oder dem Probanden gehalten oder platziert werden kann. Sowohl das Ferntest-Anzeigegerät als auch das Nahtest-Anzeigegerät können über die Steuervorrichtung von einer Bedienperson ferngesteuert werden. Die Steuervorrichtung kann dann ein Steuergerät zur Fernsteuerung aufweisen.

Der Bildschirm kann ein hintergrundbeleuchteter Bildschirm sein, wobei der Bildschirm vorzugsweise in Art eines Fernsehgeräts, Monitors oder Tablet-Computers ausgebildet sein kann. Ein Steuergerät der Steuervorrichtung kann dann auch mittels WLAN oder Bluetooth drahtlos mit dem Anzeigegerät kommunizieren. Das Steuergerät kann jedoch auch ein fest installierter Computer oder ein Laptop sein, auf dem eine Software zur Steuerung des Anzeigegeräts ausgeführt werden kann. Der hintergrundbeleuchtete Bildschirm kann ein LCD Bildschirm sein, dessen Bildschirmleuchtdichte an eine Umgebungsleuchtdichte vorzugsweise proportional anpassbar ist. Der Bildschirm kann auch beispielsweise mit linearer oder zirkularer Polarisation, oder einer anderen Einrichtung, die zur Bildtrennung nutzbar ist, ausgebildet sein.

Weiter kann vorgesehen sein, dass der Bildschirm eine Lichtquelle ausbildet. Der Bildschirm kann dann teilweise oder im Gesamten mit einer derart hohen Bildschirmleuchtdichte betrieben werden, dass alleine mit dem Bildschirm Augen des Probanden beleuchtbar sind.

Die Lichtquelle kann eine Infrarotlichtquelle sein. Mittels der Infrarotlichtquelle können Augen des Probanden ebenfalls beleuchtbar sein, insbesondere dann, wenn Sehtests bei isotopischen oder skotopischen Lichtbedingungen durchgeführt werden. So ist es aufgrund einer verminderten Umgebungshelligkeit sonst schwierig, mit einer Kameravorrichtung Augen eines Probanden zur Durchführung bestimmter Sehtests aufzunehmen. Mit der Infrarotlichtquelle können die Augen des Probanden unabhängig von einer Umgebungsbeleuchtung mit Infrarotlichtbeleuchtung mittels einer entsprechend angepassten Kameravorrichtung beziehungsweise Infrarotkamera aufgenommen werden. Vorteilhaft kann auch eine Blendung des Probanden durch die Beleuchtung der Augen mit Infrarotlicht vermieden werden. Insgesamt wird es so möglich festzustellen, ob die objektiv bei Dämmerungssehen oder Nachtsehen bestimmten Refraktionswerte von bei gleichen Beleuchtungsbedingungen und damit gleichen Pupillendurchmessern subjektiv bestimmten Refraktionswerten abweichen.

Weiter kann vorgesehen sein, die Kameravorrichtung und/oder die Infrarotlichtquelle in eine Verwahrposition im Anzeigegerät oder in einer Aufnahmeposition außerhalb des Anzeigegeräts bewegbar auszubilden. Die Kameravorrichtung und/oder die Infrarotlichtquelle kann an dem Anzeigegerät beziehungsweise Bildschirm angeordnet sein, sodass die Kamera nach Bedarf in eine Verwahrposition, beispielsweise hinter dem Bildschirm, versenkt oder für eine Kameraaufnahme in einer Aufnahmeposition neben dem Bildschirm verbracht werden kann. Eine Bewegung der Kamera von der Verwahrposition in die Aufnahmeposition und zurück kann durch eine Antriebseinheit der Kameravorrichtung erfolgen. Wenn eine vergleichsweise große Kamera verwendet wird, kann ein Umlenkprisma vorgesehen sein, sodass die Kamera platzsparend hinter dem Bildschirm angeordnet werden kann.

Darüber hinaus kann das Sehprüfsystem einen Phoropter oder eine Messbrille umfassen. Dann wird es möglich, jeweils eine subjektive Refraktion von Augen eines Probanden zu bestimmen. Auch kann der Phoropter oder die Messbrille Farbfilter oder Polarisationsfilter aufweisen, die jeweils an eine Farbdarstellung und/oder eine Polarisation des Bildschirms angepasst sind, sodass monokulare oder binokulare Sehtests durchgeführt werden können. Wenn beispielsweise ein Phoropter oder eine Messbrille mit linearer oder zirkularer Polarisation vorhanden ist, kann das Anzeigegerät des Sehprüfsystems so ausgewählt werden, dass dieses übereinstimmend mit dem Phoropter oder der Messbrille polarisiert ist. Eine Korrektur einer Polarisation, beispielsweise mit einer λ-Viertel-Folie, ist somit nicht erforderlich.

Die Lichtquelle kann aus einer Leuchtdiode ausgebildet sein, die relativ zu einem Objektiv der Kamera beziehungsweise deren optischer Achse exzentrisch angeordnet sein kann. Erst durch die exzentrische Anordnung der Leuchtdioden relativ zu der optischen Achse der Kamera wird es möglich das Auge unter einem von der optischen Achse zu der Kamera abweichenden Winkel zu beleuchten. Wenn dann die optische Achse der Kamera mit der Sehachse des Auges fluchtet, ergibt sich ein mit der Kamera durch die Pupille erfassbares Bild auf der Netzhaut. Die objektive Refraktion kann so besonders einfach auf Basis des sogenannten Fotorefraktionsprinzips bestimmt werden.

Eine Vielzahl von Leuchtdioden kann die Lichtquelle ausbilden und das Objektiv der Kamera koaxial umgeben. Auch kann vorgesehen sein, dass die Lichtquelle mehrere Infrarot (IR) Leuchtdioden umfasst. Die Lichtquellen können unmittelbar benachbart der Kamera angeordnet sein. Beispielsweise können vier Leuchtdioden neben der Kamera beziehungsweise Infrarotkamera relativ zu dieser äquidistant angeordnet sein. Prinzipiell ist es auch möglich, Leuchtidioten in einem Rahmen des Anzeigegeräts anzuordnen.

Bei dem erfindungsgemäßen Verfahren zum Überprüfen der Augen eines Probanden mit einem Sehprüfsystem weist das Sehprüfsystem ein Anzeigegerät auf, wobei mittels einer Lichtquelle einer Beleuchtungsvorrichtung des Anzeigegeräts der Augenhintergrund von Augen des Probanden beleuchtet wird, und wobei mittels einer Kamera einer Kameravorrichtung des Anzeigegeräts die Augen des Probanden aufgenommen werden, wobei mittels der Kameravorrichtung des Anzeigegeräts eine Verteilung von Licht der Lichtquelle bei der Beleuchtung des Augenhintergrundes in der Pupille des Auges des Probanden aufgenommen wird, wobei mittels einer Steuervorrichtung des Sehprüfsystems aus der Verteilung des Lichts der Lichtquelle in der Pupille eine objektive Refraktion des Auges bestimmt wird, wobei mittels eines Bildschirms des Anzeigegerätes dem Auge des Probanden Sehprüfzeichen zur subjektiven Refraktionsbestimmung unmittelbar nach der Bestimmung der objektiven Refraktion durch die Steuervorrichtung visualisiert werden. Zu den Vorteilen des erfindungsgemäßen Verfahrens wird auf die Vorteilsbeschreibung des erfindungsgemäßen Sehprüfsystems verwiesen.

Mittels der Steuervorrichtung kann das Anzeigegerät, die Lichtquelle und/oder die Kamera gesteuert werden. Die Steuervorrichtung kann weiter ein Steuergerät umfassen, welches zur Fernsteuerung des Anzeigegeräts, der Lichtquelle und/oder der Kamera verwendet werden kann. Die Bestimmung beziehungsweise Berechnung der objektiven Refraktion des Auges mittels der Steuervorrichtung kann in dem Anzeigegerät oder dem Steuergerät mittels einer Einrichtung zur Datenverarbeitung beziehungsweise eines Computers erfolgen. Wenn die Steuervorrichtung das Anzeigegerät, die Lichtquelle und die Kamera steuert, kann ein Sehtest weitestgehend automatisiert von der Steuervorrichtung durchgeführt werden.

Erfindungsgemäß werden einem ersten Schritt mittels des Sehprüfsystems die objektive Refraktion des Auges des Probanden bestimmt, und in einem zweiten Schritt mittels des Sehprüfsystems dem Auge des Probanden Sehprüfzeichen zur subjektiven Refraktionsbestimmung visualisiert, wobei der zweite Schritt unmittelbar auf den ersten Schritt folgt. Vor einer Bestimmung einer subjektiven Refraktion durch Darbietung von Sehzeichen wird dann eine objektive Refraktion bestimmt. Diese Messung kann im Wesentlichen automatisiert und auch ohne die Unterstützung einer Bedienperson erfolgen.

Die dem Probanden dargebotenen Sehprüfzeichen können daher an die objektiv gemessene Refraktion angepasst sein. Die Sehprüfzeichen können in einer Größe visualisiert werden, die an die objektiv gemessenen Refraktionswerte des Auges des Probanden angepasst ist. Mit der Bestimmung der subjektiven Refraktion erfolgt dann lediglich eine Überprüfung der objektiv gemessenen Refraktionswerte. Diese Überprüfung kann auch unter Berücksichtigung eines Pupillendurchmessers erfolgen. Da die objektiv gemessenen Refraktionswerte bei einheitlichen Lichtverhältnissen mit beispielsweise einem großen Pupillendurchmesser ermittelt wurden, kann bei der subjektiven Überprüfung der Refraktionswerte davon ausgegangen werden, dass der Pupillendurchmesser aufgrund einer nicht veränderten Umgebungsleuchtdichte im Wesentlichen gleich ist. Sofern sich zwischen der Messung der objektiven Refraktionswerte und der Messung der subjektiven Refraktionswerte die Umgebungsleuchtdichte oder auch eine Bildschirmleuchtdichte ändert, kann sich ebenfalls der Pupillendurchmesser vergleichsweise verändern, sodass sich gegebenenfalls subjektiv gemessene Refraktionswerte ergeben, die von den objektiv gemessenen Refraktionswerten abweichen. So kann es vorgesehen sein, dass ein Pupillendurchmesser beziehungsweise eine Umgebungsleuchtdichte und/oder eine Bildschirmleuchtdichte bei der Bestimmung der Refraktionswerte jeweils berücksichtigt wird.

Daher können die Sehprüfzeichen zur subjektiven Refraktionsbestimmung in Abhängigkeit der objektiven Refraktion von der Steuervorrichtung ausgewählt werden. Insbesondere können die Sehprüfzeichen in einer an den Messabstand angepassten Größe dargestellt werden, wenn ein Sehabstand beziehungsweise ein Messabstand bekannt ist. Die Darstellung der größenangepassten Sehprüfzeichen kann automatisch oder manuell über die Steuervorrichtung beziehungsweise durch eine Bedienperson erfolgen. So werden die Sehprüfzeichen immer in der tatsächlich erforderlichen Größe dargestellt und ein Zeitaufwand zur Durchführung eines Sehtests wesentlich verringert.

Insbesondere kann die Bestimmung der objektiven Refraktion mittels des Fotorefraktionsprinzips erfolgen.

Das Auge kann auch mit Leuchtdioden der Beleuchtungsvorrichtung zeitgleich oder in einer Abfolge beleuchtet werden, wobei eine jeweilige Pupillenreflektion mit der Kamera aufgenommen werden kann. Wenn die Leuchtdioden relativ zueinander beabstandet und beispielsweise koaxial zu einem Objektiv der Kamera angeordnet sind, wird es möglich aus verschiedenen Beleuchtungspositionen heraus die Netzhaut des Auges zu beleuchten, sodass eine Abfolge unterschiedlicher Pupillenreflektionen mit der Kamera aufgenommen werden kann. Diese jeweiligen Lichtverteilungen in der Pupille können dann zur Bestimmung einer verhältnismäßig genauen objektiven Refraktion des Auges benutzt werden.

Zur Bestimmung der objektiven Refraktion des Auges des Probanden kann auf einem Bildschirm des Anzeigegeräts oder an anderer Stelle des Sehprüfsystems ein Fixationsanreiz dargeboten werden. Der Fixationsanreiz kann beispielsweise ein Lichtpunkt oder ein dargestelltes Objekt sein.

Eine Sehachse des Auges des Probanden kann relativ zu einer optischen Achse der Kamera von dieser abweichend verschoben werden, dass eine tote Zone der Lichtverteilung der Pupille durch diese Blickbewegung des Auges in eine von der optischen Achse der Kamera abweichenden Richtung verschoben werden kann. Dadurch, dass dann eine Sehachse des Auges relativ zur optischen Achse der Kamera nicht fluchtet beziehungsweise wesentlich davon abweicht, wird es möglich die tote Zone der Lichtverteilung der Pupille, die bei einem Fluchten von optischer Achse der Kamera und Sehachse des Auges auftritt, durch die Blickbewegung des Auges in einer von der Kamera abweichenden Richtung zu verschieben und damit durch Aufnahme der Pupillenreflektion zu erfassen beziehungsweise eine Ametropie der toten Zone ebenfalls zu berechnen.

Optional kann ein Gradient einer Helligkeitsverteilung eines Pupillenreflexes mittels der Kameravorrichtung erfasst und mittels der Steuervorrichtung bestimmt werden. Der Gradient der Helligkeitsverteilung kann dazu genutzt werden, die objektive Refraktion des betreffenden Auges noch genauer zu bestimmen.

Mittels der Kameravorrichtung kann auch ein Pupillenabstand, ein Pupillendurchmesser, ein Messabstand, eine Kopfneigung und/oder eine Blickrichtung von Augen des Probanden erfasst und gemessen werden. Wenn ein Sehabstand des Probanden zu dem Bildschirm durch eine ortsfeste Platzierung von Bildschirm und Proband prinzipiell bekannt ist, kann mittels Bildverarbeitung aus einem mit der Kameravorrichtung beziehungsweise einer Kamera aufgenommenen Bild beider Augen des Probanden der Pupillenabstand berechnet werden. Ein Relativabstand der Pupillen kann beispielsweise zur Anpassung einer Brille oder zur Darbietung bestimmter Sehtests genutzt werden. Der Pupillendurchmesser kann in Abhängigkeit einer Umgebungsbeleuchtung ebenfalls derart gemessen werden. Sofern ein Pupillenabstand bekannt sein sollte, kann umgekehrt mittels der Bildverarbeitung aus einem mit der Kameravorrichtung aufgenommenen Bild ein Messabstand beziehungsweise Sehabstand des Probanden relativ zum Bildschirm errechnet werden. Auch können eine Kopfneigung des Probanden relativ zum Bildschirm sowie eine Blickrichtung beziehungsweise Fixation von Sehprüfzeichen erfasst werden.

So ist es auch besonders vorteilhaft, wenn mittels eines Lagesensors des Anzeigegeräts eine Position, insbesondere eine Neigung des Bildschirms relativ zu den Augen des Probanden, gemessen werden kann. Der Lagesensor kann ein gyroskopischer Sensor sein, über den eine räumliche Position beziehungsweise Lage des Bildschirms beziehungsweise der Anzeigefläche bestimmbar ist. Wenn beispielsweise eine Aufnahme der Augen des Probanden oder dessen Kopfs mittels der Kamera des Anzeigegeräts erfolgt, kann unter Einbeziehung eines bekannten Pupillenabstands leicht eine Neigung des Bildschirms relativ zu den Augen errechnet werden. Über den Bildschirm kann dann auch angezeigt werden, dass der Bildschirm relativ zu den Augen geneigt ist und beispielsweise kein Sehtest durchgeführt werden kann. Auch können über den Bildschirm Informationen zur richtigen Ausrichtung des Bildschirms relativ zu den Augen des Probanden ausgegeben werden. Der Proband ist so gegebenenfalls selbst in der Lage, den Bildschirm relativ zu seinen Augen in die für einen Sehtest erforderliche Position zu bringen.

Mittels der Kamera kann auch eine kontinuierliche Blickerfassung von Augen des Probanden erfolgen. Demnach kann ein Fixationspunkt der Augen auf einer Anzeigefläche des Bildschirms errechnet werden. Dies ist dann möglich, wenn eine Blickrichtung der Augen des Probanden erfasst wird. So kann im Rahmen von Sehtests untersucht werden, inwieweit der Proband monokular oder binokular dargebotene Sehprüfzeichen dynamisch verfolgt.

Weitere Ausführungsformen des Verfahrens ergeben sich aus den auf den Vorrichtungsanspruch 1 rückbezogenen Unteransprüchen.

Nachfolgend wird eine bevorzugte Ausführungsform der Erfindung unter Bezugnahme auf die beigefügten Zeichnungen näher erläutert.

Es zeigen:
- **Fig. 1:**: eine Vorderansicht eines Sehprüfsystems;
- **Fig. 2:**: eine schematische Darstellung eines Beleuchtungsstrahlengangs des Sehprüfsystems;
- **Fig. 3:**: eine schematische Darstellung eines Beobachtungsstrahlengangs des Sehprüfsystems;
- **Fig.4:**: eine grafische Darstellung einer Lichtverteilung in einer Pupille relativ zu einer Ametropie.

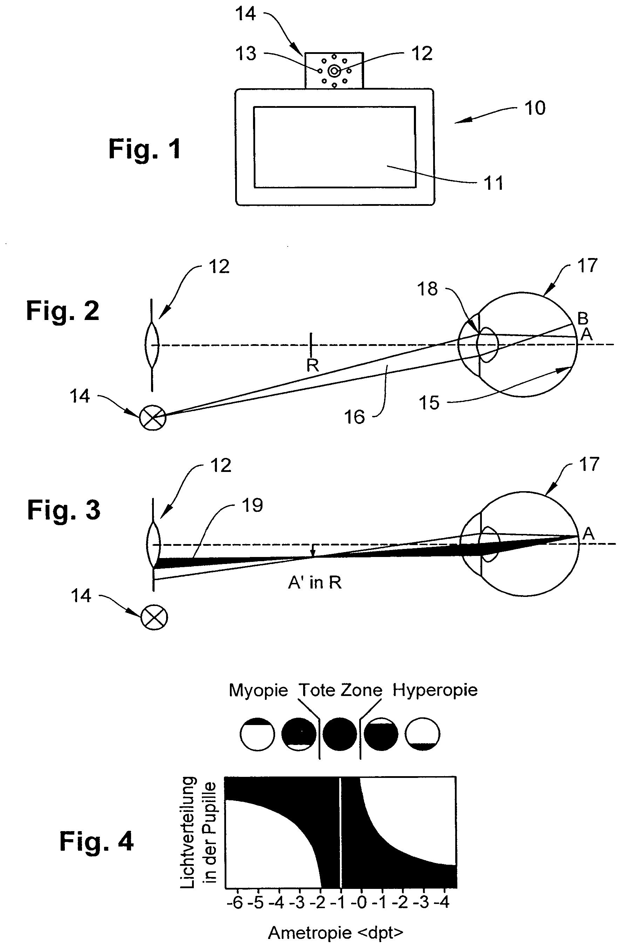

Die **Fig. 1** zeigt eine Vorderansicht eines Sehprüfsystems 10 mit einem Bildschirm 11, einer Kamera 12 und einer Vielzahl von InfrarotLeuchtdioden 13, die relativ zur Kamera 12 koaxial angeordnet sind. Zur Überprüfung der Sehfähigkeit eines Probanden ist es regelmäßig erforderlich, durch Darstellung von Sehprüfzeichen auf einem Bildschirm sich der Ametropie des betroffenen Auges des Probandes iterativ anzunähern und einen Refraktionswert auf diesem Wege, beispielsweise mit einer Messbrille oder einem Phoropter, subjektiv zu bestimmen. Auch ist es möglich zunächst gesondert vorab einen objektiven Refraktionswert zu messen, bevor eine eingegrenzte subjektive Refraktionsmessung erfolgt. Die objektive Refraktionsmessung wird beispielsweise mit einem Autorefraktometer unabhängig von den bekannten subjektiven Sehprüfsystemen vergleichsweise genau durchgeführt.

Mit dem Sehprüfsystem 10 ist nun vorgesehen, das Sehprüfsystem 10 zunächst zur objektiven und unmittelbar anschließend zur subjektiven Refraktionsbestimmung zu verwenden. Die Bestimmung der objektiven Refraktion erfolgt auf Basis des sogenannten Fotorefraktionsprinzips, bei dem, wie in **Fig. 2** dargestellt, mittels einer Lichtquelle 14 eine Netzhaut 15 beziehungsweise ein Augenhintergrund beleuchtet wird. Dabei ergibt sich bei dem in der **Fig. 2** beispielhaft gezeigten Beleuchtungsstrahlengang 16 eines myopen Auges 17 ein unscharfes Bild BA auf der Netzhaut 15. Dieses Bild BA wird durch die Pupille 18 des Auges 17 gemäß dem in **Fig. 3** gezeigten Beobachtungsstrahlengang 19 mit der Kamera 12 aufgenommen. Diese aufgenommene Lichtverteilung der Pupille 18 beziehungsweise Pupillenreflektion ist jeweils unterschiedlich in Abhängigkeit der vorliegenden Ametropie des Auges 17, wie die Darstellung in **Fig. 4** zeigt. Bei einer Überprüfung der Augen 17 eines Probanden wird daher zunächst das Auge 17 mit den Leuchtdioden 13 zeitgleich oder in einer Abfolge beleuchtet und eine Pupillenreflektion mit der Kamera 12 aufgenommen. Aus der Pupillenreflektion kann dann mittels einer hier nicht dargestellten Steuervorrichtung, welche zur Steuerung des Sehprüfsystems 10, des Bildschirms 11 und der Kamera 12 dient, eine Ametropie beziehungsweise objektive Refraktion des Auges 17 mittels Bildverarbeitung errechnet werden. Unmittelbar nach dieser gegenüber einem Autorefraktometer vergleichsweise ungenauen Bestimmung der objektiven Refraktion können dann dem Probanden auf dem Bildschirm 11 Sehprüfzeichen dargeboten werden, die an die objektiv gemessene Refraktion angepasst sind und somit eine schnellere subjektive Refraktionsbestimmung ohne Wechsel des Sehprüfsystems 10 oder einer Messabstandsänderung ermöglichen.

Zur Bestimmung der objektiven Refraktion kann auf dem Bildschirm 11 oder an anderer Stelle des Sehprüfsystems 10 ein Fixationsanreiz dargeboten werden, derart, dass dann eine Sehachse 21 des Auges 17 relativ zu einer optischen Achse 22 der Kamera 20 nicht fluchtet beziehungsweise wesentlich davon abweicht. Dann wird es auch möglich die tote Zone der Lichtverteilung der Pupille nach **Fig. 4****,** die bei einem Fluchten von optischer Achse 22 der Kamera 20 und Sehachse 21 des Auges 17 auftritt, durch die Blickbewegung des Auges 17 in eine von der Kamera 20 abweichende Richtung zu verschieben und damit durch Aufnahme der Pupillenreflektion zu erfassen beziehungsweise eine Ametropie der toten Zone ebenfalls zu berechnen.

## Patentansprüche

1. Sehprüfsystem (10) zum Überprüfen der Augen eines Probanden, aufweisend ein Anzeigegerät mit dem zumindest einem Auge (17) des Probanden Sehprüfzeichen zur subjektiven Refraktionsmessung visualisierbar sind, wobei das Anzeigegerät eine Kameravorrichtung mit einer Kamera (12) aufweist, mittels der Augen des Probanden aufnehmbar sind, wobei das Anzeigegerät eine Beleuchtungsvorrichtung mit einer Lichtquelle (14) aufweist, mittels der der Augenhintergrund des Probanden beleuchtbar ist, wobei mittels der Kameravorrichtung des Anzeigegeräts eine Verteilung von Licht der Lichtquelle bei der Beleuchtung des Augenhintergrunds in der Pupille (18) des Auges des Probanden aufnehmbar ist, wobei das Sehprüfsystem eine Steuervorrichtung aufweist, mittels der aus der Verteilung des Lichts der Lichtquelle in der Pupille eine objektive Refraktion des Auges bestimmbar ist, wobei das Anzeigegerät einen Bildschirm (11) umfasst, wobei die Steuervorrichtung geeignet ist, den Bildschirm so zu steuern, dass die Sehprüfzeichen für die subjektive Refraktionsmessung unmittelbar nach der Bestimmung der objektiven Refraktion durch die Steuervorrichtung visualisiert werden können.

2. Sehprüfsystem nach Anspruch 1,
**dadurch gekennzeichnet,**
**dass** das Anzeigegerät, die Kamera (12) und die Lichtquelle (14) in einem gemeinsamen Gehäuse des Sehprüfsystems (10) angeordnet sind.

3. Sehprüfsystem nach Anspruch 1 oder 2,
**dadurch gekennzeichnet,**
**dass** das Anzeigegerät ein ortsfestes Ferntest-Anzeigegerät ist, dessen Anzeigeflächengröße für Sehtests mit einem Sehabstand von 3 m bis 10 m ausgebildet ist und/oder ein mobil handhabbares Nahtest-Anzeigegerät ist, dessen Anzeigeflächengröße für Sehtests mit einem Sehabstand von 10 cm bis 3 m ausgebildet ist.

4. Sehprüfsystem nach einem der vorangehenden Ansprüche,
**dadurch gekennzeichnet,**
**dass** der Bildschirm (11) ein hintergrundbeleuchteter Bildschirm ist, wobei der Bildschirm in Art eines Fernsehgeräts, Monitors oder Tablet-Computers ausgebildet ist.

5. Sehprüfsystem nach Anspruch 4,
**dadurch gekennzeichnet,**
**dass** der Bildschirm (11) die Lichtquelle ausbildet.

6. Sehprüfsystem nach einem der vorangehenden Ansprüche,
**dadurch gekennzeichnet,**
**dass** die Lichtquelle (14) eine Infrarotlichtquelle ist.

7. Sehprüfsystem nach einem der vorangehenden Ansprüche,
**dadurch gekennzeichnet,**
**dass** die Lichtquelle (14) aus einer Leuchtdiode (13) ausgebildet ist,
die relativ zu einem Objektiv der Kamera (12) bzw. deren optischer Achse (22) exzentrisch angeordnet ist.

8. Sehprüfsystem nach Anspruch 7,
**dadurch gekennzeichnet,**
**dass** eine Vielzahl von Leuchtdioden (13) die Lichtquelle (14) ausbilden und das Objektiv der Kamera (12) koaxial umgeben.

9. Verfahren zum Überprüfen der Augen eines Probanden mit einem Sehprüfsystem (10), aufweisend ein Anzeigegerät, wobei mittels einer Lichtquelle (14) einer Beleuchtungsvorrichtung des Anzeigegeräts der Augenhintergrund von Augen (17) des Probanden beleuchtet wird, und wobei mittels einer Kamera (12) einer Kameravorrichtung des Anzeigegeräts die Augen des Probanden aufgenommen werden, wobei mittels der Kameravorrichtung des Anzeigegeräts eine Verteilung von Licht der Lichtquelle bei der Beleuchtung des Augenhintergrundes in der Pupille (18) des Auges des Probanden aufgenommen wird, wobei mittels einer Steuervorrichtung des Sehprüfsystems aus der Verteilung des Lichts der Lichtquelle in der Pupille eine objektive Refraktion des Auges bestimmt wird,
**dadurch gekennzeichnet,**
**dass** mittels eines Bildschirms (11) des Anzeigegerätes dem Auge des Probanden Sehprüfzeichen zur subjektiven Refraktionsbestimmung unmittelbar nach der Bestimmung der objektiven Refraktion durch die Steuervorrichtung visualisiert werden.

10. Verfahren nach Anspruch 9,
**dadurch gekennzeichnet,**
**dass** mittels der Steuervorrichtung das Anzeigegerät, die Lichtquelle (14) und/oder die Kamera (12) gesteuert werden.

11. Verfahren nach einem der Ansprüche 9 oder 8,
**dadurch gekennzeichnet,**
**dass** die dem Probanden dargebotenen Sehprüfzeichen an die objektiv gemessene Refraktion angepasst sind.

12. Verfahren nach einem der Ansprüche 9 bis 11,
**dadurch gekennzeichnet,**
**dass** die Sehprüfzeichen in Abhängigkeit der objektiven Refraktion von der Steuervorrichtung ausgewählt werden.

13. Verfahren nach einem der Ansprüche 8 bis 11,
**dadurch gekennzeichnet,**
**dass** die Bestimmung der objektiven Refraktion mittels des Fotorefraktionsprinzips erfolgt.

14. Verfahren nach einem der Ansprüche 8 bis 12,
**dadurch gekennzeichnet,**
**dass** das Auge (17) mit Leuchtdioden (13) der Beleuchtungsvorrichtung zeitgleich oder in einer Abfolge beleuchtet wird, wobei eine jeweilige Pupillenreflexion mit der Kamera (12) aufgenommen wird.

15. Verfahren nach einem der Ansprüche 8 bis 13,
**dadurch gekennzeichnet,**
**dass** zur Bestimmung der objektiven Refraktion auf einem Bildschirm (11) des Anzeigegeräts oder an anderer Stelle des Sehprüfsystems (10) ein Fixationsanreiz dargeboten wird.

16. Verfahren nach einem der Ansprüche 8 bis 14,
**dadurch gekennzeichnet,**
**dass** eine Sehachse (21) des Auges (17) des Probanden relativ zu einer optischen Achse (22) der Kamera (12) von dieser abweichend verschoben wird, derart, dass eine tote Zone der Lichtverteilung der Pupille (18) durch diese Blickbewegung des Auges (17) in eine von der optischen Achse (22) der Kamera (12) abweichenden Richtung verschoben wird.

17. Verfahren nach einem der Ansprüche 8 bis 15,
**dadurch gekennzeichnet,**
**dass** ein Gradient einer Helligkeitsverteilung eines Pupillenreflexes mittels der Kameravorrichtung erfasst und mittels der Steuervorrichtung bestimmt wird.

## Claims

1. An eye examining system (10) for examining a subject's eyes, the eye examining system having a display device by means of which eye examination symbols are made visible to at least one of the subject's eyes (17) for a subjective refraction measurement, the display device comprising a camera device having a camera (12) by means of which the subject's eyes can be recorded, the display device comprising an illumination device having a light source (14) by means of which the subject's ocular fundus can be illuminated, a light distribution of the light source being able to be recorded in the pupil (18) of the subject's eye by means of the camera device of the display device when illuminating the ocular fundus, the eye examining system comprising a control apparatus by means of which an objective refraction of the eye can be determined from the light distribution in the pupil, the display device comprising a monitor (11), the control apparatus being suitable for controlling the monitor in such a manner that the eye examination symbols can be made visible for the subjective refraction measurement directly after the objective refraction has been determined by the control apparatus.

2. The eye examining system according to claim 1,
**characterized in that**
the display device, the camera (12) and the light source (14) are arranged in a shared housing of the eye examining system (10).

3. The eye examining system according to claim 1 or 2,
**characterized in that**
the display device is a stationary display device for testing farsightedness, whose display surface size is designed for conducting eye examinations at a seeing distance of 3 m to 10 m, and/or is a display device for testing nearsightedness for mobile use, whose display surface size is designed for conducting eye examinations at a seeing distance of 10 cm to 3 m.

4. The eye examining system according to any one of the preceding claims,
**characterized in that**
the monitor is a backlit monitor (11), the monitor preferably being designed as a type of television, monitor or tablet computer.

5. The eye examining system according to claim 4,
**characterized in that**
the monitor (11) forms the light source.

6. The eye examining system according to any one of the preceding claims,
**characterized in that**
the light source (14) is an infrared light source.

7. The eye examining system according to any one of the preceding claims,
**characterized in that**
the light source (14) is formed by a light-emitting diode (13) which is eccentrically arranged relative to a lens of the camera (12) or rather its optical axis (22).

8. The eye examining system according to claim 7,
**characterized in that**
a plurality of light-emitting diodes (13) form the light source (14) and coaxially surround the lens of the camera (12).

9. A method for examining a subject's eyes with an eye examining system (10), the eye examining system having a display device, the ocular fundus of the subject's eyes (17) being illuminated by means of a light source (14) of an illumination device of the display device, and the subject's eyes being recorded by means of a camera (12) of a camera device of the display device, a light distribution of the light source being recorded in the pupil (18) of the subject's eye by means of the camera device of the display device when illuminating the ocular fundus, an objective refraction of the eye being determined from the light distribution of the light source in the pupil by means of a control apparatus of the eye examining system,
**characterized in that**
eye examining symbols are made visible to the subject's eye by means of a monitor (11) of the display device for the subjective refraction determination directly after the objective refraction has been determined by the control apparatus.

10. The method according to claim 9,
**characterized in that**
the display device, the light source (14) and/or the camera (12) are controlled by means of the control apparatus.

11. The method according to any one of the claims 9 or 8,
**characterized in that**
the eye examination symbols shown to the subject are adjusted to the objectively measured refraction.

12. The method according to any one of the claims 9 to 11,
**characterized in that**
the eye examination symbols are chosen by the control apparatus in dependence of the objective refraction.

13. The method according to any one of the claims 8 to 11,
**characterized in that**
the objective refraction is determined by means of the photo refraction principle.

14. The method according to any one of the claims 8 to 12,
**characterized in that**
the eye (17) is illuminated by light-emitting diodes (13) of the illuminating device simultaneously or sequentially, a corresponding pupil reflection being recorded by the camera (12).

15. The method according to any one of the claims 8 to 13,
**characterized in that**
a fixation stimulus is presented on a monitor (11) of the display device or at a different position of the eye examining system (10) for determining the objective refraction.

16. The method according to any one of the claims 8 to 14,
**characterized in that**
a visual axis (21) of the subject's eye (17) is displaced varyingly to an optical axis (22) of the camera (12) relative thereto in such a manner that a blind spot of the pupil's (18) light distribution is displaced by this visual movement of the eye (17) in a direction varying to the optical axis (22) of the camera (12).

17. The method according to any one of the claims 8 to 15,
**characterized in that**
a gradient of a brightness distribution of a pupil reflex is detected by means of the camera device and is determined by means of the control apparatus.

## Revendications

1. Système de test (10) de la vision pour examiner les yeux d'un patient, le système de test de la vision ayant un dispositif d'affichage au moyen duquel des signaux de test de l'oeil sont rendus visibles à au moins un oeil (17) du patient pour une mesure de réfraction subjective, le dispositif d'affichage comprenant un dispositif de caméra ayant une caméra (12) au moyen de laquelle les yeux du patient peuvent être enregistrés, le dispositif d'affichage comprenant un dispositif d'illumination ayant une source de lumière (14) au moyen de laquelle le fond de l'oeil du patient peut être illuminé, une distribution de la lumière de la source de lumière pouvant être enregistrée dans la pupille (18) de l'oeil du patient au moyen du dispositif de caméra du dispositif d'affichage quand le fond de l'oeil est illuminé, le système de test de la vision comprenant un appareil de commande au moyen duquel une réfraction objective de l'oeil peut être déterminée dans la pupille à partir de la distribution de la lumière de la source de lumière, le dispositif d'affichage comprenant un écran (11), l'appareil de commande étant susceptible de commander l'écran de telle manière que les signaux de test de l'oeil sont rendus visibles pour la mesure de réfraction subjective directement après que la réfraction objective a été déterminée par l'appareil de commande.

2. Système de test de la vision selon la revendication 1,
**caractérisé en ce que**
le dispositif d'affichage, la caméra (12) et la source de lumière (14) sont disposés dans un boîtier commun du système de test (10) de la vision.

3. Système de test de la vision selon la revendication 1 ou la revendication 2,
**caractérisé en ce que**
le dispositif d'affichage est un dispositif d'affichage stationnaire pour le test de l'hypermétropie, dont taille de la surface d'affichage est configurée pour effectuer des tests de vision à une distance de vue de 3 m à 10 m, et/ou est un dispositif d'affichage mobile pour le test de myopie, dont taille de la surface d'affichage est configurée pour effectuer des tests de vision à une distance de vue de 10 cm à 3 m.

4. Système de test de la vision selon l'une quelconque des revendications précédentes,
**caractérisé en ce que**
l'écran (11) est un écran rétroéclairé, l'écran étant configuré de préférence de manière d'un téléviseur, d'un écran ou d'une tablette numérique.

5. Système de test de la vision selon la revendication 4,
**caractérisé en ce que**
l'écran (11) forme la source de lumière.

6. Système de test de la vision selon l'une quelconque des revendications précédentes,
**caractérisé en ce que**
la source de lumière (14) est une source de lumière infrarouge.

7. Système de test de la vision selon l'une quelconque des revendications précédentes,
**caractérisé en ce que**
la source de lumière (14) est faite d'une diode électroluminescente (13) qui est disposée excentriquement par rapport à un objectif de la caméra (12) et/ou à son axe optique (22).

8. Système de test de la vision selon la revendication 7,
**caractérisé en ce**
**qu'**une pluralité de diodes électroluminescentes (13) forme la source de lumière (14) et encercle coaxialement l'objectif de la caméra (12).

9. Procédé pour examiner les yeux du patient avec un système de test (10) de la vision, le système de test de la vision ayant un dispositif d'affichage, le fond de l'oeil de yeux (17) du patient étant illuminé au moyen d'une source de lumière (14) d'un dispositif d'illumination du dispositif d'affichage, et les yeux du patient étant enregistrés au moyen d'une caméra (12) d'un dispositif de caméra du dispositif d'affichage, une distribution de la lumière de la source de lumière étant enregistrée dans la pupille (18) de l'oeil du patient au moyen du dispositif de caméra du dispositif d'affichage quand le fond de l'oeil est illuminé, une réfraction objective de l'oeil étant déterminée dans la pupille à partir de la distribution de la lumière de la source de lumière au moyen d'un appareil de commande du système de test de la vision,
**caractérisé en ce que**
des signaux de test de l'oeil sont rendus visibles à l'oeil du patient au moyen d'un écran (11) du dispositif d'affichage pour la détermination de la réfraction subjective directement après que la réfraction objective a été déterminée par l'appareil de commande.

10. Procédé selon la revendication 9,
**caractérisé en ce que**
le dispositif d'affichage, la source de lumière (14) et/ou la caméra (12) sont commandés au moyen de l'appareil de commande.

11. Procédé selon l'une quelconque des revendications 9 ou la revendication 8,
**caractérisé en ce que**
les signaux de test de l'oeil montés au patient sont adaptés à la réfraction objective mesurée.

12. Procédé selon l'une quelconque des revendications 9 à 11, **caractérisé en ce que**
les signaux de test de l'oeil sont choisis par l'appareil de commande en fonction de la réfraction objective.

13. Procédé selon l'une quelconque des revendications 8 à 11,
**caractérisé en ce que**
la réfraction objective est déterminée au moyen du principe de la photoréfraction.

14. Procédé selon l'une quelconque des revendications 8 à 12,
**caractérisé en ce que**
l'oeil (17) est illuminé par des diodes électroluminescentes (13) du dispositif d'illumination simultanément ou en série, une réflexion correspondante de la pupille étant enregistrée par la caméra (12).

15. Procédé selon l'une quelconque des revendications 8 à 13,
**caractérisé en ce**
**qu'**un stimulus de fixation est présenté à un écran (11) du dispositif d'affichage ou à un endroit différent du système de test (10) de la vision pour la détermination de la réfraction objective.

16. Procédé selon l'une quelconque des revendications 8 à 14,
**caractérisé en ce**
**qu'**un axe visuel (21) de l'oeil (17) du patient est déplacé de manière variante d'un axe optique (22) de la caméra (12) par rapport auquel de telle manière qu'une zone de silence de la distribution de la lumière de la pupille (18) est déplacée par ce mouvement de l'oeil (17) dans une direction de manière variante de l'axe optique (22) de la caméra (12).

17. Procédé selon l'une quelconque des revendications 8 à 15,
**caractérisé en ce**
**qu'**un gradient de la distribution de la luminosité d'un réflexe de la pupille est détecté au moyen du dispositif de caméra et est déterminé au moyen de l'appareil de commande.
